Europäisches Patentamt

European Patent Office

Office européen des brevets

(11) Publication number: **0 087 735**
A2

# EUROPEAN PATENT APPLICATION

(21) Application number: ·83101714.0

(22) Date of filing: 23.02.83

(51) Int. Cl.³: **C 12 N 15/00**
**A 61 K 39/108**

(30) Priority: 25.02.82 JP 30059/82

(43) Date of publication of application:
07.09.83 Bulletin 83/36

(84) Designated Contracting States:
BE CH DE FR GB IT LI NL SE

(71) Applicant: Takeda Chemical Industries, Ltd.
27, Doshomachi 2-chome
Higashi-ku Osaka, 541(JP)

(72) Inventor: Yokota, Takeshi
7-1, Narashinodia 3-chome
Funabashi Chiba 274(JP)

(72) Inventor: Yamamoto, Tatsuo
38-2, Oaza-Kaname Oh-hocho
Tsukuba-gun Ibarak 300-26(JP)

(74) Representative: von Kreisler, Alek, Dipl.-Chem. et al,
Deichmannhaus am Hauptbahnhof
D-5000 Köln 1(DE)

(54) Recombinant DNA, microorganism transformed therewith and their use.

(57) The present invention provides a novel recombinant DNA containing an IAF structural gene and free of an ST structural gene, a transformed microorganism containing an IAF structural gene, an LTA* structural gene and an LTB structural gene and which is free of an ST structural gene, and the use of said transformed microorganism as a prophylactic agent against toxigenic bacterial infections.

EP 0 087 735 A2

- 1 -

SPECIFICATION

Recombinant DNA, Microorganism Transformed Therewith, and Their Use

This invention relates to a novel recombinant DNA, a microorganism transformed therewith and their use. More particularly, the invention relates to a recombinant DNA which contains an IAF structural gene and which is free of an ST structural gene, to transformed microorganism which contains said IAF structural gene, an LTA* structural gene and an LTB structural gene, and which is free of an ST structural gene and to the use of said transformed microorganism as a prophylactic agent against toxigenic bacterial infections.

Traveler's diarrhea, which is a nuisance to those who travel within developing countries such as Asia and the region covering from North America to Central and South America, is a kind of bacterial diarrhea, and may cause severe symptoms, and bacterial diarrhea in young animals such as piglets and calves is a severe problem in the livestock industry because it causes deaths or, in case of survival, poor growth rates. It is known that the main factor causing such bacterial diarrhea is exotoxins released by toxigenic strains of Escherichia coli. Many strains of toxigenic Escherichia coli produce a heat-labile enterotoxin (LT; molecular weight about 80,000) which is a protein and loses its toxicity upon heating at 60°C for 30 minutes, a heat-stable enterotoxin (ST; molecular weight 2,000-5,000) which retains its toxicity even after heat treatment, and an intestine-adhering factor (IAF), which is a protein necessary for adhesion of the cell membrane to the intestinal mucosa [Infection and Immunity, 12, 656-667 (1975), Infection and Immunity, 19, 1021-1030 (1978) and Journal of Medical Microbiology, 3, 387-401 (1970)]. LT is bound, by means of subunit B (LTB), to ganglioside $GM_1$ of the epithelial cell of the intestinal mucosa and then subunit A (LTA) forces a passage through the lipid layer of the cytoplasmic membrane and causes an increase in cyclic AMP (cAMP) level, whereby a change in permeability of the cytoplasmic membrane results and induces diarrhea through release of large amounts of water and bicarbonate ion from the cell. On the other hand, ST causes an increase in intracellular cyclic GMP (cGMP) level and thereby causes diarrhea [Proceedings of the Fifteenth Joint Conference on Cholera, 142-147, U.S. NIH Publication No. 80-2003 (February 1980) and Proceedings of the Fifteenth Joint Conference on Cholera, 373-388, U.S. NIH Publication No. 80-2003 (February 1980)]. The genes for the expression of these pathogenicity factors all reside on plasmids.

An enterotoxin (ENT) plasmid carrying the structural genes for LT and ST has first been demonstrated in toxigenic Escherichia coli strains of porcine mass diarrhea origin [Journal of General Microbiology, 52, 319-334 (1968)]. Subsequent DNA analysis has revealed that it is a circular DNA (cccDNA) having a molecular weight of about 40-60 megadaltons. The genes for the toxins on the plasmid have also been identified. It is known that the ST gene in toxigenic Escherichia coli strains of porcine origin is a transposon and is transposable from the ENT plasmid to other plasmids [Nature, 277, 453-456 (1979)].

The present inventors analyzed plasmids of Escherichia coli H 10407 (O 78:H 11) of diarrheal patient origin, one of toxigenic Escherichia coli strains for which the relationship between the plasmid and the expression of pathogenicity is most fully understood, and found a plasmid producing the two enterotoxins (LT and ST) [Journal of Bacteriology, 143, 652-660 (1980)]. They also cloned both the LT and ST genes and analyzed the gene structures, and created recombinant DNAs respectively encoding LTA* and LTB free of ST activity [Journal of Bacteriology, 145, 850-860 (1981) and Journal of Bacteriology, 148, 983-987 (1981)].

It is known to stock farmers from experience that oral administration of wild bacterial strains derived from animals with diarrhea to sows or cows prior to delivery is effective against diarrhea in young animals. However, much care is required in handling wild strains because of possible pathogenicity. Moreover, the use of wild strains may pose such problems as environmental pollution through fecal excretion and instability in effectiveness.

If a microorganism free of toxicity but having an antibody-producing ability is created by transformation with a recombinant DNA, coding for the production of both IAF and extracellular nontoxic polypeptides, which is created by genetic manipulation of virulence genes originating from some toxigenic strain of Escherichia coli, such microorganism can be expected as a safe and highly useful live vaccine.

As a result of intensive research in such technical background, the present inventors have succeeded in creating a recombinant DNA containing the IAF structural gene but lacking the ST structural gene by separating the IAF gene from the plasmid on which both the IAF and ST structural genes are carried and have thus opened the way to production of a live vaccine for prophylaxis of bacterial diarrheal infection through cultivating a microorganism transformed with said recombinant DNA.

Thus, the present invention provides: (1) a recombinant DNA which contains an IAF structural gene and which is free of an ST structural gene, (2) a transformed microorganism which contains said IAF structural gene, an LTA* structural gene and an LTB structural gene, and which is free of an ST structural gene and (3) the use of said transformed microorganism as a prophylactic agent against toxigenic bacterial infections.

The genes involved in the expression of pathogenicity of toxigenic strains of Escherichia coli are carried by plasmids, namely a plasmid carrying the IAF- and ST-encoding genes and a plasmid carrying the LT- and ST-encoding genes. They are nontransmissible plasmids. The essential feature of the present invention consists in creation of DNAs free of the ST gene by removal of the gene for ST which is toxic and almost incapable of inducing antibody production, further in preparation of a DNA containing the gene for LTA* which is a polypeptide which induces antibody production but is almost free of the toxicity of LTA and a DNA containing the gene for LTB, and in creation of a microorganism with these DNAs introduced therein by transformation.

The IAF structural gene of this invention includes, for example, ones possessed by toxigenic Escherichia coli strains of diarrheal human or animal origins, such as a colonization factor antigen (CFA) structural gene of human origin, a K88 antigen gene of porcine origin and a K99 antigen gene of bovine origin.

Brief Description of the Drawings

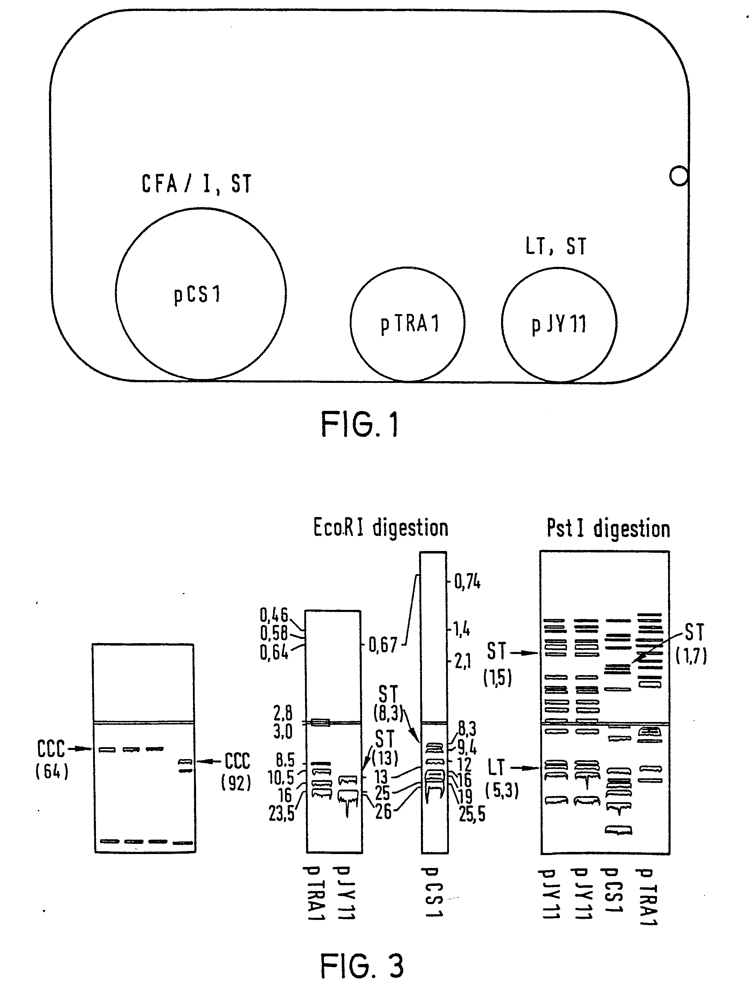

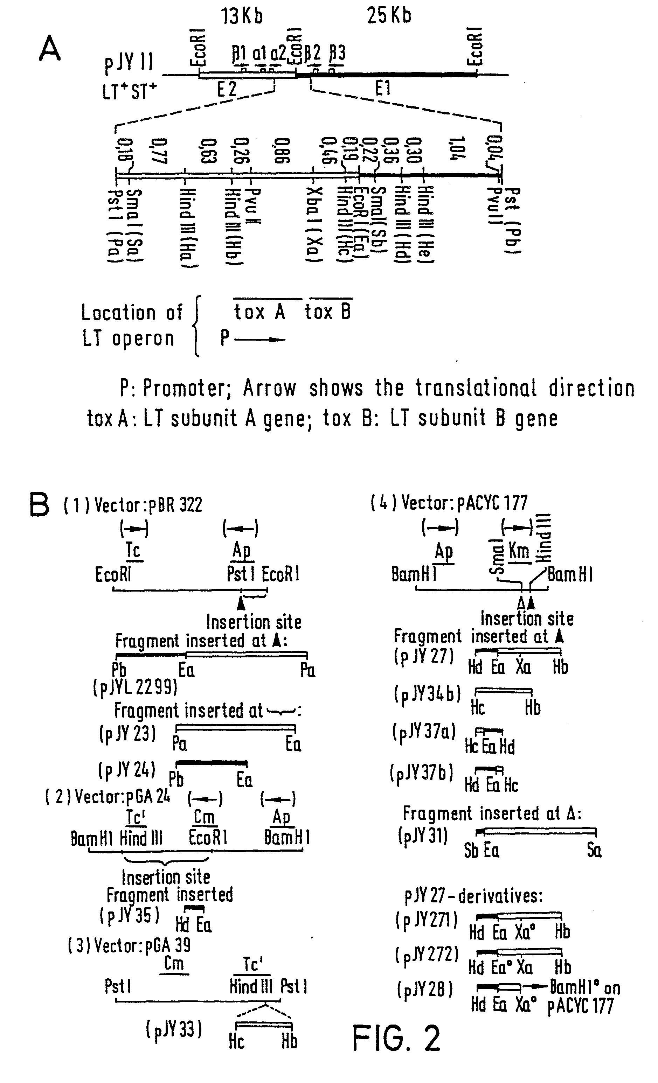

Fig. 1 is a schematic representation of plasmids of patient-derived Escherichia coli H 10407 (0 78:H 11), Fig. 2 is the physical mapping of LT gene-carrying recombinant DNAs, and Fig. 3 shows the agarose slab gel electrophoresis patterns of pCS1, pJY11 and pTRA1 DNAs after digestion with restriction enzymes.

The invention is further illustrated in the following by taking as an example the case where plasmids prepared from diarrheal patient-derived toxigenic Escherichia coli H 10407 (078:H 11) are used.

Escherichia coli H 10407 is a known toxigenic strain [Infection and Immunity, 12, 656-667(1975) and Journal of Bacteriology, 143, 652-660(1980)] and is also on deposit in the Institute for Fermentation, Osaka (Deposit No. IFO-14223).

As shown in Fig. 1, H 10407 carries at least four species of plasmids. Among them, pCS1 is a nontransmissible plasmid having a molecular weight of about $62 \times 10^6$ [Infection and Immunity, 12, 656-667(1975)]. The inventors have confirmed that pCS1 contains both the genes for CFA/I (which is a type of CFA) and for ST.

pJY11 is a nontransmissible plasmid having a molecular weight of about $42 \times 10^6$ and contains the genes for ST and LT [Journal of Bacteriology, 143, 652-660(1980), Journal of Bacteriology, 145, 850-860(1981) and Journal of Bacteriology, 148, 983-987, (1981)].

pTRA1 is a transmissible plasmid having a molecular weight of about $42 \times 10^6$ and having, in addition to its self-transmissibility, a function of causing transmission of pCS1 and pJY11 to other bacterial strains. The remaining one is a plasmid having a molecular weight of about $4 \times 10^6$; it is not related to the expression of pathogenicity and its functions are unknown.

An Escherichia coli strain carrying pCS1 alone or pJY11 alone can be prepared as follows. RP4 (drug-resistant plasmid) is transmitted to Escherichia coli H 10407 by conjugation, so that the ampicillin transposon (Tn1) on RP4 is transferred to pTRA1. Since pTRA1::Tn1 (pJY12), like pTRA1, is capable of transmitting pCS1 and pJY11 to other cells, the conjugation results in ampicillin resistance and production of CFA or LT.

Adequate selection of strains gives strains carrying two plasmid species (pJY12, pCS1) or (pJY12, pJY11). By selecting out the strain which has lost ampicillin resistance spontaneously, there can be obtained a strain carrying pCS1 or pJY11 alone.

Preparation of LT toxoid-producing plasmid

The LT holotoxin is composed of two subunits (A and B). The subunit B (LTB) is capable of adhering to the receptor on the epithelial cell of the intestinal mucosa. Following this adhesion of LTB, the subunit A (LTA) acts on adenylate-cyclase in the membrane to exhibit enzymatic activity (toxin activity). Therefore, LTB and a toxin activity-deficient, antigenic LTA derivative (LTA*) both can be LT toxoids. The LT operon of pJY11 has the structure shown in Fig. 2A. Using recombinant DNA technique, this LT operon region on pJY11 is first cloned as a PstI fragment into the pBR322 at its PstI site, so that pJYL2299 is obtained (Fig. 2B). Further, from the pJYL2299 DNA as a material, the following toxoid-producing plasmids are prepared by using recombinant DNA technique [Journal of Bacteriology, 145, 850-860(1981) and Journal of Bacteriology, 148, 983-987(1981)].

## Preparation of LTB-producing recombinant plasmid

Since LTB gene (toxB) has no promoter, an LTB-producing plasmid is prepared for the expression of toxB by insertion of a fragment carrying toxB gene into a vector downstream from the translational portion on the operon thereof. Typical examples of such plasmids are pJY24 (tetracycline-resistant, LTB-producing), pJY35 (ampicillin-resistant, LTB-producing), pJY37a (ampicillin-resistant, LTB-producing) and pJY26 (ampicillin-resistant, LTB-producing) [Fig. 2B, Journal of Bacteriology, 145, 850-860(1981) and Journal of Bacteriology, 148, 983-987(1981)].

## Preparation of LTA-producing plasmid

A plasmid producing a toxin activity-deficient derivative (LTA*, molecular weight 17,000) of LTA (molecular weight 26,800) is prepared by insertion of the LT promoter and toxA gene region (segment), with deletion of a C-terminus portion, into a vector. Since an LT promoter exists on toxA gene, these can be inserted into a vector at any desired site. A typical example of the thus-obtained plasmid is pJY34b (ampicillin-resistant, LTA*-producing) [Fig. 2 and Journal of Bacteriology, 148, 983-987(1981)].

## Preparation of CFA gene-containing plasmid

pCS1 contains not only a CFA gene but also an ST gene. The ST produced by it showed a stronger activity than the ST produced by pJY11 when tested in suckling mice. A CFA-producing plasmid is prepared by subjecting pCS1 to PstI or EcoRI digestion for removal of the ST gene. A typical example of such plasmids is pJY40 (kanamycin-resistant, CFA-producing).

Preparation of vaccine strain

The present inventors have found that LTB is released from the cell efficiently, that, in the presence of an LTA-producing plasmid alone, LTA is not released from the cell at all and that LTB plays an important role in the release of LTA [Journal of Bacteriology, 150, 1482-1484(1982)]. A highly useful live vaccine can be produced based on these findings. Thus, a CFA-producing plasmid, an LTA*-producing plasmid and an LTB-producing plasmid are introduced into one and the same strain of Escherichia coli by transformation, and there can be obtained a live vaccine which produces the colonization factor antigen (CFA) for adhesion to the intestinal mucosa cell and toxoids (LTA* and LTB). A drug-sensitive live vaccine can also be obtained by preparing a plasmid having a selective marker other than a drug-resistance and introducing into Escherichia coli by transformation. The phage resistance gene, lactose metabolism gene, tryptophan synthesis gene and other amino acid synthesis genes, for instance, can be used as such selective markers.

In the above explanation, the case where an Escherichia coli live vaccine is produced by using the plasmids of patient-derived Escherichia coli H 10407 has been described. In addition, it is also possible to clone another colonization factor antigen (CFA/II) gene [Infection and Immunity, 21, 638-647(1978)] possessed by another patient-derived toxigenic strain of Escherichia coli in place of the above-mentioned CFA/I gene. Furthermore, while construction of three plasmids respectively containing the CFA-, LTA*- and LTB- producing genes has been described in the above, one and the same plasmid may contain two or three of said three genes.

Furthermore, an Escherichia coli live vaccine for veterinary use can be produced by cloning an IAF- producing gene possessed by a toxigenic Escherichia coli strain of diarrheal animal origin, such as the K88 antigen gene of porcine origin [Journal of Bacteriology, 145, 920-925(1981)] or the K99 antigen gene of bovine origin [Infection and Immunity, 29, 1125-1133(1980)], in the reported manner and using the cloning gene in place of the above-mentioned CFA/I gene.

The methods of assaying the components released from the cell are described below. The LT activity was assayed on the basis of morphological change in the Chinese hamster ovary-derived CHO-K1 cell [Infection and Immunity, 10, 320-327(1974)]. The ST activity was assayed by the suckling mouse method [Journal of Infectious Diseases, 125, 407-411(1972)]. The detection of CFA (intestine-adhering protein) was performed based on the human erythrocyte agglutination reaction in the presence of mannose [Infection and Immunity, 18, 330-337(1977)]. The latex method was used for the detection of the LT subunits (LTA or LTB) proteins.

The microorganisms to be transformed include Escherichia coli, Salmonella species, Klebsiella species, Yersinia species, other enteric bacteria and Pseudomonas aeruginosa. Since cholera toxin productivity-deficient strains have been found among Vibrio cholerae strains of natural origin [Infection and Immunity, 32, 661-667(1981)], live vaccines for cholera can also be produced by transformation of these non-toxin-producing Vibrio cholerae strains with the plasmids in accordance with the present invention.

The microorganisms transformed in accordance with the invention can be cultivated in the same manner as the wild strain. As well as a live vaccine containing the wild strain [Proceedings International Pig Veterinary Society J.2 (1976)], the live cells obtained by cultivation of the transformants can be administered orally as a live vaccine to warm-blooded animals (pig, cattle, sheep, poultry, humans, etc.), especially to mammals, for production of antibodies in said animals, whereby the animals can be protected from toxigenic bacterial infections. The protection of such animals from toxigenic bacterial infections can also be achieved parenterally by injecting a processed matter of the culture by removing live cells therefrom, for example a culture filtrate, to said animals.

In the following, the recombinant DNAs containing the CFA, LTA* and LTB genes, respectively, in accordance with the invention and the microorganisms transformed with said DNAs are further described based on an example. Said example, however, is by no means limitative of the present invention.

## Example

(1)    Preparation of CFA/I-producing recombinant plasmid pCS1 (molecular weight about $62 \times 10^6$) is obtained by the method of Evans et al. [Infection and Immunity, 12, 656-667(1975)]. Then pCS1::Tn5 is obtained by causing transposition of kanamycin transposon (Tn5) to said pCS1. The pCS1::Tn5 DNA is partially digested with PstI and then annealed with a ligase. Since the annealed product contains a plasmid DNA lacking a PstI fragment containing the 1.7 Kb ST gene (cf. Fig. 3), said DNA is introduced into Escherichia coli by transformation and CFA$^+$ strains are selected using kanamycin resistance as a marker. A plasmid (molecular weight about $30 \times 10^6$, pJY40) exhibiting kanamycin resistance and CFA/I productivity is obtained from these strains.

(2)    Preparation of LTB-producing recombinant plasmid
The method of Yamamoto et al. [Journal of Bacteriology, 145, 850-860(1981)] is used. The pJYL 2299 DNA is cleaved with PstI and EcoRI and the 2 kb EcoRI·PstI fragment (in Fig. 2B, Ea-Pb) containing toxB gene is inserted into pBR322 (vector) at the EcoRI·PstI site to give pJY24 (tetracycline-resistant).

(3)     Preparation of LTA*-producing recombinant plasmid

The method of Yamamoto et al. [Journal of Bacteriology, 148, 983-987(1981)] is followed.  The pJYL2299 DNA is cleaved with HindIII, and the 1.58 kb HindIII fragment (in Fig. 2B, Hb-Hc) having the LT promoter and the toxA gene region residue lacking 0.25 - 0.3 kb portion counted from the codon coding for C-terminus is inserted into pACYC177 (vector) at the HindIII site to give pJY34b (ampicillin-resistant).

(4)     Preparation of vaccine strain (1)

The three plasmids, pJY40, pJY34b and pJY24, are introduced into one and the same strain of Escherichia coli by the transformation procedure [Proceedings of the National Academy of Sciences of the United States of America, 69, 2110-2114(1972)].  The resulting Escherichia coli strain has drug resistance.

(5)     Preparation of vaccine strain (2)

The restriction enzyme-generated fragments obtained from pJY40, pJY34b and pJY24 and containing the LT promoter as well as the toxB region, toxA portion and CFA/I gene, respectively, and an Rts1 fragment carrying the phage resistance gene (containing the $T_4$ resistance gene) are purified with restriction enzymes, and the single-stranded DNA portions at the both ends of each fragment are rendered double-stranded with DNA polymerase. These fragments are ligated in sequence to the restriction site in the drug resistance gene portion of pBR322 with $T_4$ DNA ligase.  Finally, there is prepared a recombinant plasmid exhibiting phage resistance and producing LTB, LTA* and CFA/I.  This plasmid is introduced into Escherichia coli by transformation using the phage resistance as a marker.

The following references, which are referred to for their disclosures at various points in this application, are incorporated herein by reference.

Infection and Immunity, 12, 656-667(1975)

Infection and Immunity, 19, 1021-1030(1978) Journal of Medical Microbiology, 3, 387-401(1970) Proceedings of the Fifteenth Joint Conference on Cholera, 142-147, U.S. NIH Publication No. 80-2003 (February 1980) Proceedings of the Fifteenth Joint Conference on Cholera, 373-388, U.S. NIH Publication No. 80-2003 (February 1980) Journal of General Microbiology, 52, 319-334(1968) Nature, 277, 453-456(1979) Journal of Bacteriology, 143, 652-660(1980)

Journal of Bacteriology, 145, 850-860(1981) Journal of Bacteriology, 148, 983-987(1981) Journal of Bacteriology, 150, 1482-1484(1982) Infection and Immunity, 21, 638-647(1978) Journal of Bacteriology, 145, 920-925(1981) Infection and Immunity, 29, 1125-1133(1980) Infection and Immunity, 10, 320-327(1974) Journal of Infectious Diseases, 125, 407-411(1972) Infection and Immunity, 32, 661-667(1981) Proceedings of the National Academy of Sciences of the United States of America, 69, 2110-2114(1972) Proceedings International Pig Veterinary Society, J.2(1976) Infection and Immunity, 18, 330-337(1977)

What is claimed is:

(1)    A recombinant DNA which contain an IAF structural gene and which is free of an ST structural gene.

(2)    A recombinant DNA according to Claim 1, wherein the IAF structural gene is a CFA or K88 antigen or K99 antigen structural gene.

(3)    A recombinant DNA according to Claim 1, wherein the IAF structural gene encodes CFA/I of an enteropathogenic Escherichia coli.

(4)    A recombinant DNA according to Claims 1 to 3, which further contains an LTA* structural gene and/or an LTB structural gene.

(5)    A recombinant DNA according to Claim 3, which is of about $30 \times 10^6$ daltons and which comprises an kanamycin-resistant gene.

(6)    A transformed microorganism which contains an IAF structural gene, an LTA* structural gene and an LTB structural gene, and which is free of an ST structural gene.

(7)    A transformed microorganism according to Claim 6, wherein the IAF structural gene is a CFA or K88 antigen or K99 antigen structural gene.

(8)    A transformed microorganism according to Claim 6, wherein the IAF structural gene encodes CFA/I of an enteropathogenic Escherichia coli.

(9)    A transformed microorganism according to Claims
6 to 8, which is Escherichia coli.

(10)    A prophylactic agent against toxigenic bacterial
infections which contains a culture or its processed
matter of a transformed microorganism, which contains
an IAF structural gene, an LTA* structural gene and
an LTB structural gene and which is free of an ST structural
gene.

(11)    A prophylactic agent against toxigenic bacterial
infections according to Claim 10, the IAF structural
gene is a CFA or K88 antigen or K99 antigen structural
gene.

(12)    A method of producing a recombinant DNA which
contains an IAF structural gene and which is free of
an ST structural gene, which method comprises eliminating
with a restriction enzyme an ST structural gene from
a DNA containing both an IAF structural gene and an
ST structural gene.

(13)    A method of producing a recombinant DNA according
to Claim 12, wherein the IAF structural gene is a CFA
or K88 antigen or K99 antigen structural gene.

(14)    A method of producing a recombinant DNA according
to Claim 12, wherein the IAF structural gene encodes
CFA/I of an enteropathogenic Escherichia coli.

(15)    A method of producing a transformed microorganism
which contains an IAF structural gene, an LTA* structural
gene and an LTB structural gene, and which is free
of an ST structural gene, which method comprises introducing
above-mentioned three structural genes into one and the same strain of a microorganism by the transformation procedure.

(16)   A method of producing a transformed microorganism according to Claim 15, wherein the IAF structural gene is a CFA or K88 antigen or K99 antigen structural gene.

(17)   A method of producing a transformed microorganism according to Claim 15, wherein the IAF structural gene encodes CFA/I of an enteropathogenic Escherichia coli.

(18)   A method of producing a transformed microorganism according to Claims 15 to 17, wherein the microorganism is Escherichia coli.

(19)   A method of producing a prophylactic agent against toxigenic bacterial infections, which method comprises growing a transformed microorganism which contains an IAF structural gene, an LTA* structural gene and an LTB structural gene, and which is free of an ST structural gene, and if desired, processing the resulting culture broth.

(20)   A method of producing a prophylactic agent against toxigenic bacterial infections according to Claim 19, wherein the IAF structural gene is a CFA or K88 antigen or K99 antigen structural gene.

FIG.1

EcoRI digestion

Pst I digestion

FIG. 3

FIG. 2